(19)

Europäisches Patentamt

European Patent Office

Office européen des brevets

(11) EP 2 083 871 B1

(12) **EUROPEAN PATENT SPECIFICATION**

(45) Date of publication and mention
of the grant of the patent:
**21.03.2018 Bulletin 2018/12**

(21) Application number: **07827056.8**

(22) Date of filing: **12.11.2007**

(51) Int Cl.:
**G01T 1/161** (2006.01)    **A61B 6/00** (2006.01)

(86) International application number:
**PCT/IB2007/054578**

(87) International publication number:
**WO 2008/059426 (22.05.2008 Gazette 2008/21)**

(54) **DETERMINATION OF A DISTRIBUTION OF RADIOACTIVE AGENTS IN A SUBJECT**

BESTIMMUNG EINER VERTEILUNG RADIOAKTIVER WIRKSTOFFE IN EINEM SUBJEKT

DETERMINATION D'UNE DISTRIBUTION D'AGENTS RADIOACTIFS DANS UN SUJET

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **14.11.2006 EP 06123988**
**09.02.2007 EP 07102024**

(43) Date of publication of application:
**05.08.2009 Bulletin 2009/32**

(73) Proprietors:
• **Koninklijke Philips N.V.**
**5656 AE Eindhoven (NL)**
Designated Contracting States:
**AT BE BG CH CY CZ DK EE ES FI FR GB GR HU
IE IS IT LI LT LU LV MC MT NL PL PT RO SE SI
SK TR**
• **Philips Intellectual Property & Standards GmbH**
**20099 Hamburg (DE)**
Designated Contracting States:
**DE**

(72) Inventors:
• **GEORGI, Jens-Christoph**
**5656 AE Eindhoven (NL)**
• **SCHWEIZER, Bernd**
**5656 AE Eindhoven (NL)**
• **VON BUSCH, Heinrich**
**5656 AE Eindhoven (NL)**

(74) Representative: **van Velzen, Maaike Mathilde**
**Philips Intellectual Property & Standards**
**High Tech Campus 5**
**5656 AE Eindhoven (NL)**

(56) References cited:
**WO-A-02/086449      US-A1- 2003 125 616**

**Description**

FIELD OF THE INVENTION

The present invention relates to a method for determining a biodistribution of radioactive agents in a subject.

BACKGROUND OF THE INVENTION

The treatment of neoplastic processes with internal radiation such as targeted radiotherapy typically involves incorporation of radioactive agents in the human body. These substances are subject to a spatial distribution within the body and exponential decay of activity over time.

To get information about the spatial distribution of the labels within the body the emitted radiation of the radioactive agents is measured over time. This can be done by corresponding devices or methods, e.g. Positron Emission Tomography (PET), Single Photon Emission Computed Tomography (SPECT), planar scintigraphy, and blood samples; Sgouros G: Dosimetry of Internal Emitters, J Nucl Med 2005; 46:18-27 *and the Publications of the Medical Internal Radiation Dose (MIRD) Committee (http://interactive.snm.org/index.cfm?PageID=1372&RPID=2199),* and used for calculating the dose distribution within the patient in different organs. By measuring the time-activity-curve for a particular organ, i.e. the emitted radiation for a particular organ over time, it is possible to calculate the amount of radioactive decays (i.e. the radioactivity) in this respective organ from this respective label. Since the type of the isotope is known (type of radiation, energy, charge etc), this integrated activity (integrated over time) can be converted to a local dose, absorbed in this organ. The result thereof is then used for evaluation of the treatment planning or treatment monitoring and dose verification.

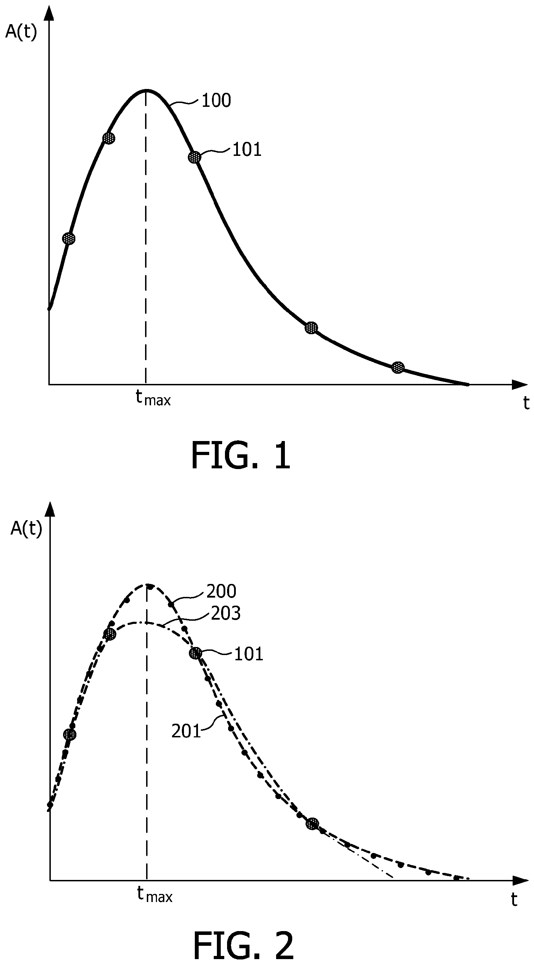

The disadvantage of current dosimetry or monitoring methods like scintigraphy or emission tomography, such as SPECT or PET, is that due to the high costs of these procedures a maximum of about three to five examinations per patient is available. This temporal under sampling can easily result in substantial errors in the estimation of the time-activity-curve and therefore in the dosimetry calculation as well. As different organs show different pharmacokinetic behavior, this means that for different organs, imaging is not necessarily performed at the time-points that most characteristically define the respective time-activity-curves, which typically extends over several days. Therefore, highly valuable information can get lost and the dose calculation can be inaccurate. This is illustrated graphically in Fig. 1 showing an example of a theoretical time activity curve indicated by a solid line 100 and a possible distribution of data points collected using e.g. SPECT, shown as circles 101 for a particular organ or tissue. As this Fig. depicts, due to the lack of data points when methods like SPECT are being used the maximum emission from the organ at time $t_{max}$ is not captured by the SPECT. The only way to calculate the doses is to initially fit through these 3-5 data points and subsequently calculate the integral based on the resulting fitting curve. It is obvious that the fitting curve can easily indicate a substantially wrong behavior and deviate considerably from the true curve and this will result in inaccurate dosimetry calculation.

One way to avoid multiple nuclear imaging procedures is to measure the whole-body dose with a single, non-imaging detector placed in front of the patient. While this type of measurement can be performed rather quickly and is inexpensive compared to the SPECT and PET methods, all information about the spatial distribution of radioactivity within the body is lost. It is therefore very difficult to provide an accurate therapy planning.

WO 02/086449 A2 discloses methods, systems, devices and computer program products that monitor in vivo detected radiation in a target localized site within a subject, over a selected time period, to do one or more of: (a) quantify a radiation dose received at a local site; (b) assess bioreceptiveness to a particular treatment time or type; (c) evaluate the pharmacokinetics of a radiolabeled analyte corresponding to a non-radiolabeled analyte; (d) monitor or evaluate metabolic activity; or (e) evaluate a tumor prior to or after a therapeutic treatment.

US 2003/0125616 A1 discloses disposable single-use external dosimeters for use in radiation therapies. The disposable single-use dosimeter patch comprises a body holding a circuit with at least one MOSFET and an external reader contact region thereon, the at least one MOSFET having an associated threshold voltage that changes when exposed to radiation, the body comprising opposing upper and lower primary surfaces. A dosage amount can be obtained using an external portable dose-reader configured to make electrical contact with the patch by physically engaging with the contact region on the patch to obtain voltage threshold data corresponding to the dose amount of radiation exposure it is exposed to in use.

BRIEF DESCRIPTION OF THE INVENTION

The object of the present invention is to overcome the above mentioned drawbacks by providing a simplified and inexpensive method for determining very precisely and in a user friendly manner the spatial distribution of absorbed dose values of radioactivity for different organs or tissues. The present invention relates to a method for determining a biodistribution of radioactive agents in a subject as defined in claim 1.

In that way, valuable information is provided for e.g. research and approval phase of new pharmaceuticals. For example, for a new chemo-therapy drug against cancer that has a radiolabel attached to it, the system could be implemented to provide information about how this drug is metabolized in the body, e.g. for research purposes or in an FDA approval trial. Later, after the drug is approved, it might be administered only in the unlabeled (i.e. in the absence of the radioactive isotope) version. The determined biodistribution may also be used for calculating or extrapolating the dose that will be absorbed in the different tissues in a succeeding therapy step. A further problem overcome by the present invention is that due to the simplicity of the detector system the subject does no longer have to stay at or come to the hospital for the measurement. Another advantage realized by the present invention is the adaptation of the measuring rate to the pharmacokinetic behavior of the tissues, since this will ensure sufficiently high measuring rate when required, i.e. it is ensured that the calculation of the biodistribution will be much more accurate. Therefore more convincing information is provided when e.g. planning a radiation treatment with regard to evaluating the maximum allowable dose for internal radiotherapy without risking that normal or risk tissues will be damaged.

The term tissue includes target tissues, e.g. tumor parts, tumor, inflamed tissue or any other tissue intended to be targeted by the radioactive agent, and/or risk tissues, e.g. healthy organ parts or organs. The term subject refers to according to the present invention a human being, an animal or any other kind of biological species.

In one example, each detector from the two or more detectors is selected from the group of:

- MOSFET-based detectors arranged to sense gamma radiation in an energy range of 100-550keV,
- diode-based detectors,
- film-based detectors,
- Thermal Luminescent Detectors (TLD),
- gel-based detectors, and
- a combination of the above.

In one example, the processor is an external processor, the system further comprising a transmitter for transmitting the separate radiation data sets via a communication channel to the external processor.

In that way, the whole processing is performed externally from the subject. This results in lower weight and smaller dimensions of the detectors, making the system easy to use. The communication channel may e.g. include a wireless communication network or wired communication channel, e.g. optical fibers and the like.

In one example, the method further uses a receiver for receiving the transmitted separate radiation data sets.

In one embodiment, the determined biodistribution of the radioactive agents is used for estimating a dose distribution of succeeding radioactive therapeutic agents in the subject.

Thus, from the determined biodistribution (i.e. time-activity-curves), it is possible to calculate or extrapolating the dose that will be absorbed in the different tissues in succeeding therapy step with e.g. beta- or alpha emitting radioactive therapeutic agents. Thus, this information can assist a doctor or other skilled person to select the amount of doses during therapeutic treatment without risking damaging risk tissues.

In one embodiment, the radioactive agents are the radioactive agents are adapted to be attached or associated to pharmaceuticals so that the biodistribution of the radioactive agents reflect a biodistribution of the pharmaceuticals within the subject.

Thus, the information about how this drug is metabolized in the body will be highly valuable for research purposes or in an FDA approval trial, where e.g. the drug is will be administered only in the unlabeled version, i.e. in the absence of the radioactive agents.

According to the present invention, adapting the at least one measuring rate to the pharmacokinetic behavior of the localized tissues comprises adapting the at least one measuring rate to activity dynamics of the localized tissues.

Since high activity dynamics is reflected in faster changes in emitted radiation the measuring rate or the count-rate will be increased accordingly such that all relevant data is acquired, whereas where the activity dynamics is low the changes in emitted radiation will be slower and thereby the measuring rate will be slower. It is thereby ensured that the relevant data points that characterize the time-activity curve are captured. As an example, if the emitted radiation changes very rapidly over a first time interval up to a maximum value it might be preferred to measure the emitted radiation over this time interval very rapidly to capture the accurate shape of time activity curve. However, in the subsequent time interval where the emitted radiation decreases relative slowly, the measuring rate may be reduced without jeopardizing that information get lost. Such an adjustment of the sensors can be done manually or automatically.

Particularly, adapting the at least one measuring rate to the pharmacokinetic behavior of the localized tissues comprises using a single measuring rate that is adapted to the pharmacokinetic behavior of the tissue having the highest activity dynamics.

Since the pharmacokinetic behavior of the tissues can be very different, the adaptation of the measuring rate to the tissue having the highest activity dynamics results in that no relevant data points will be neglected. The result thereof is a very precise calculation in the biodistribution of the imaging agent within the tissues.

In one embodiment, processing the data sets comprises:

- applying a fitting process to each respective data set from the separate radiation data sets, the fitting process resulting in a time-activity curve associated to the each respective data set, and subsequently
- determining the integral of the time-activity curve for the each respective data set.

In one embodiment, the measuring is performed until a count rate of the radiation emitted from the radioactive agents has fallen below a pre-defined threshold value. In that way, an automatic "stop" feature is provided meaning that all collected data below this threshold level are not of particular relevance and therefore the measurement for that particular detector can be stopped or suspended.

BRIEF DESCRIPTION OF THE DRAWINGS

Embodiments of the invention will be described, by way of example only, with reference to the drawings, in which

Fig. 1 shows an example of a theoretical time activity curve indicated by a solid line and a possible distribution of data points collected using prior art techniques,
Fig. 2 illustrates graphically an example the intensity A(t) of measured radiation data emitted from a tissue over time t,
Fig. 3 shows a number of radiation detectors attached to the skin of a patient,
Fig. 4 shows a system according to the present invention for determining spatial distribution of radioactive agents in a subject, and
Fig. 5 shows a flowchart of a method according to the present invention.

DESCRIPTION OF EMBODIMENTS

Treatments of neoplastic processes with internal radiation such as targeted radiotherapy typically involve incorporation of pre-defined amount of imaging agents in the body of a subject. Such agents typically emit gamma rays such as Indium ($^{111}$In), where the quantity of the labels that is used may be around 5mCi, e.g. the imaging version of the agent Zevalin®. These labels are subject to spatial distribution within the body and exponential decay of activity over time. By measuring the emitted radiation for different tissues over time information about the biodistribution in the tissues is provided. Based on this information, it is e.g. possible to calculate or extrapolate the dose that will be absorbed in the tissues (organ parts or organs as an example) in the succeeding therapy with a beta- or alpha emitting isotope in internal therapy planning. Examples of such beta- or alpha emitting isotopes are $^{90}$Y or $^{211}$At. In the case of $^{90}$Y-Zevalin, the therapeutic version of the drug Zevalin, a dose of 0.4mCi/kg body weight (but not more than 32mCi) is administered. Thus, this information is highly relevant for evaluating maximum allowable doses for internal radiotherapy planning so that risk tissues will not be damaged. Also, the biodistribution in the tissues gives valuable information in research and approval phase of new pharmaceuticals where e.g. a new chemo-therapy drug against cancer that has such radiolabels attached to it. The biodistribution of the agents provides then information about how this drug is metabolized in the body, e.g. for research purposes or in an FDA approval trial. Later, after the drug is approved, it might be administered only in the unlabeled version.

Fig. 2 illustrates graphically an example of the intensity curve *A(t),* derived from measured radiation data emitted from a tissue over time t. The risk tissues could, according to the present invention form healthy organ parts or whole organs, and the target tissues might refer to a tumor. For example if radiolabeled antibodies are used as therapeutic agent, the main risk organ is the bone marrow. If radiolabeled peptide are used the main risk organ is the kidney. The data points are measured by at least one radiation detector placed on or attached to the skin of the subject, adjacent to the tissue. The attaching to the skin could e.g. be done by using adhesive strips taped onto the detectors. Since the radiation coming from the body is being measured it is of course preferred that no intermediate material is placed between the skin and the detectors.

The detectors are set up to adapt the measuring rate to the pharmacokinetic behavior of the tissues to ensure that sufficient amount of data points are being captured. Due to the high data collection rate the large number of data points reflect very accurately the actual shape of the time-activity-curve 201 from this particular tissue.

Since the time-activity-curve detected by a sensor will not only correspond to the pharmacokinetics in a single organ or region-of-interest (ROI), but will also be influenced by other organs, the sensor signal $s_j(t)$ of the different ROIs *i* is given by:

$$s_j(t) = \sum_i w_{ij} \cdot a_i(t). \qquad (1)$$

The weighing factors $w_{ij}$ describe the influence of activity $a_i$ in ROI $i$ on the signal of sensor $j$. Each detector corresponds to one ROI, the remaining background is covered by one additional ROI as well. The matrix $w_{ij}$ can be chosen e.g. as a function of the distance $r_{ij}$ form ROI $i$ to sensor $j$. Thus, if factor matrix $w_{ij}$ is known and the signal from the sensors $s_j(t)$ is measured, the activity $a_i(t)$ in organ $i$ can be calculated.

The $a_i(t)$ may further on be used to generate artificial, interpolated SPECT-or PET-images based on the one or two conventional imaging studies that would still be needed. The activity visible in the nuclear images in a region corresponding to the "viewing field" of a detector can be scaled with the $A_i(t)$-curve of the respective detector to yield an estimated activity image for later time points. Different detectors can influence different regions of the nuclear image; and with a suitable interpolation mechanism between these regions, synthetic PET-or SPECT images for additional time points can be obtained. This higher number of images can be used in an image-based planning software for targeted radiotherapies with the goal to increase the accuracy of the plan.

The weighting matrix $w_{ij}$ (defined above) may be determined by the following methods:

I. the $w_{ij}$ matrix can be calculated based on a physical model of the patient using a simple point source model for ROI $i$:

$$w_{ij}(r_{ij}) = C \cdot \exp(-\mu \cdot r_{ij})/(r_{ij} \cdot r_{ij}) \qquad (2)$$

Here, C is an overall scaling factor and $\mu$ is the mean attenuation coefficient of the detected radiation in the patient's tissue. $\mu$ as well as $r_{ij}$ can be derived from previously acquired CT images. If the number of detectors is equal to or larger than the number of relevant ROIs, the set of linear equations for all detectors in eq. (1) can be inverted in order to obtain the pharmacokinetic behavior $a_i(t)$ of the agent in the ROIs. Further, to get a more reliable relationship between measured activity at the detector and activity at the region of interest, calibration procedures with a suitable phantom may be performed. The geometry for this model could be based on e.g. representative standard patients or patient specific data coming e.g. from CT images.

II. the $w_{ij}$ matrix can be calculated based on numerical simulations of a patient model using e.g. Monte Carlo Methods. The geometry for this model could be based on e.g. representative standard patients or patient specific data coming e.g. from CT or MR images.

III. the $w_{ij}$ matrix can directly be calculated for the different time points one has the SPECT or PET images for. The $s_j$ are measured, the $a_i$ are known from the image data, so the $w_{ij}$ can be calculated for the different timepoints using eq. (1). Afterwards the resulting matrices can be averaged to one final $w_{ij}$.

The calculation of the dose is preferably done by calculating the integral of the time activity curve, e.g. as described by the Medical Internal Radiation Dose (MIRD) Committee and e.g. reported in *"G. Sgouros, The Journal of Nuclear Medicine, Vol. 46, No. 1 (Suppl), January 2005, p. 18s-27s"* and the publications of the MIRD committee *(http://interactive.snm.org/index.cfm?PageID=1372&RPID=2199).* Thus, the result of the integral determines the biodistribution within the subject and therefore the dose that will be absorbed by the tissues in the succeeding therapeutic treatment where e.g. beta-and alpha emitting isotopes are used. As disclosed by G. Sgouros et.al. the time-activity-curve is integrated over time resulting in total number of decays divided by the injected activity. This results in the so-called "residence time", given by the equation:

$$\tau = \frac{1}{A_{inj}} \int a_i(t)dt$$

where $a_i(t)$ is the time activity curve in the ROI $i$ and $A_{inj}$ is the injected activity of the diagnostic/imaging agent. The residence time is the starting point for dose calculations based on e.g. Monte-Carlo-Simulations or the so-called S-value approach. The S-value matrix describes the crosstalk between source and target-regions taking into account the geometrical properties of the subject and the physical properties of the radiation, e.g. the S value matrix states how much dose is absorbed in a target organ per accumulated activity in a source organ. Thus, if the residence time is known it is possible to calculate the absorbed internal dose for a specific isotope and a specific patient model. Thus, the term "residence time" may just as well be applied instead of the term "dose" and be converted to the term dose.

The data points from Fig. 1 are shown here for comparison to reflect one of the important aspects of the present invention, namely to enhance the measuring rate to reflect precisely the biodistribution in the tissues. As mentioned in the background, prior methods that are capable of providing spatial information about the doses distribution within the body are e.g. positron emission tomography (PET), single photon emission computed tomography (SPECT) methods that are based on capturing 3D nuclear images of the body. The processing of the images results in spatial data indicating the amount of emitted radiation from various organs for a single time point (i.e. at the time when the imaging takes places). Fig. 2 shows four data points 101 for a single organ resulting from four imaging procedures (other organs would accordingly be associated with four data points). This low number of data points is due to the high costs following these imaging procedures. In today's praxis a patient typically receives only between three to four examinations. Due to the limited number of data points, the resulting time-activity-curve can be very misleading, which obviously can lead to an inaccurate estimate of the biodistribution. Since this biodistribution is the main parameter for determining the maximum allowable doses for radiotherapy planning, the risk of causing a permanent damage in risk tissues can be dramatically increased. This can also lead to that the doses for the therapeutic treatment becomes too low, since the doctor or other skilled person wants to be on the safe side.

In one embodiment, the data points are collected at fixed time interval e.g. every ½ hour or every hour, but this time interval is preferably adjustable e.g. depending on the organs being measured since the different tissues can have different pharmacokinetic behavior. Accordingly, for some tissues it might be preferable to detect the emitted radiation data with higher rate than for other tissues. The detectors could also be arranged to collect the emitted radiation data with a fixed measuring rate, e.g. every ½ hour, or every 10 minutes, or every minute, if it is ensured that the resulting data set reflects very accurately the pharmacokinetic behavior of the tissue having the highest activity dynamics.

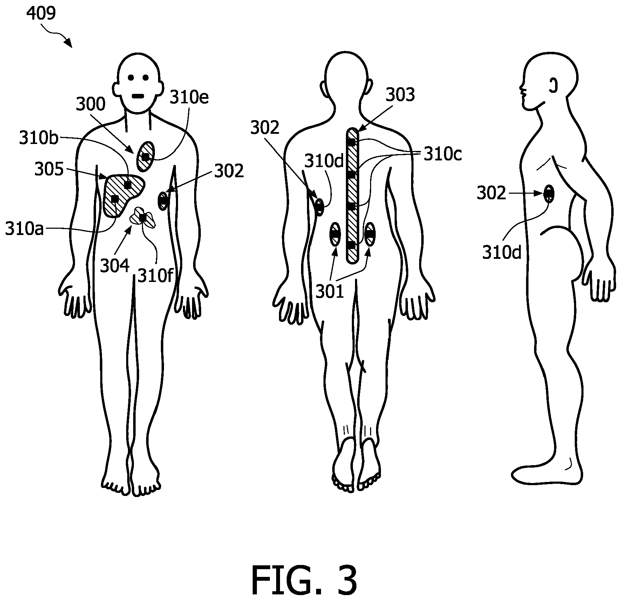

Fig. 3 shows where a number of radiation detectors 310a-310f have been attached to the skin of a subject 409, as shown here a human being. In this particular example the localized tissue areas form the heart 300, the kidneys 301, the spleen 302 and the spinal cord 303 and the target volume 304. As shown here, the detectors are attached substantially directly above these organs or the tumor. As an example, one detector 310e is placed directly above the heart 300, one detector is placed directly above each respective kidney 301, one detector 310d is placed directly above the spleen 302, four detectors 310c are placed along the spinal cord 303, two detectors 310a,b are placed along the liver 305 and one detector 310f is placed directly above the target volume 304.

As previously mentioned, prior to the therapeutic treatment the biodistribution of the radioactive imaging agents in the subject must be determined in order to determine the dose distribution of succeeding radioactive therapeutic agents, or to provide information to be used in research and/or approval phase of new pharmaceuticals where e.g. a new drug has such radio label attached to it.

As shown here, the biodistribution of the imaging agents in heart 300, the kidney 301, the spleen 302, the bone marrow 303, the liver 305 and the target volume 304 is determined. This may be done to plan the succeeding internal therapy step with e.g. alpha-and beta emitting isotopes. In one embodiment, the functioning of the detectors is such that they autonomously perform cumulative measurements of local activity by detecting emitted radiation from the imaging agents in the organs/risk area 300-305, where the measurements preferably start right after the injection of the radioactive imaging agents and continue until the activity falls below a certain threshold level which can be after several days, e.g. 10 days is estimated for the heart 300, whereas e.g. four dose values are estimated for the spinal cord where each value is associated to each respective detector 310c resulting in a local dose distribution within the 303. It is also possible to combine these four measurements to a single value of mean dose in the spinal cord.

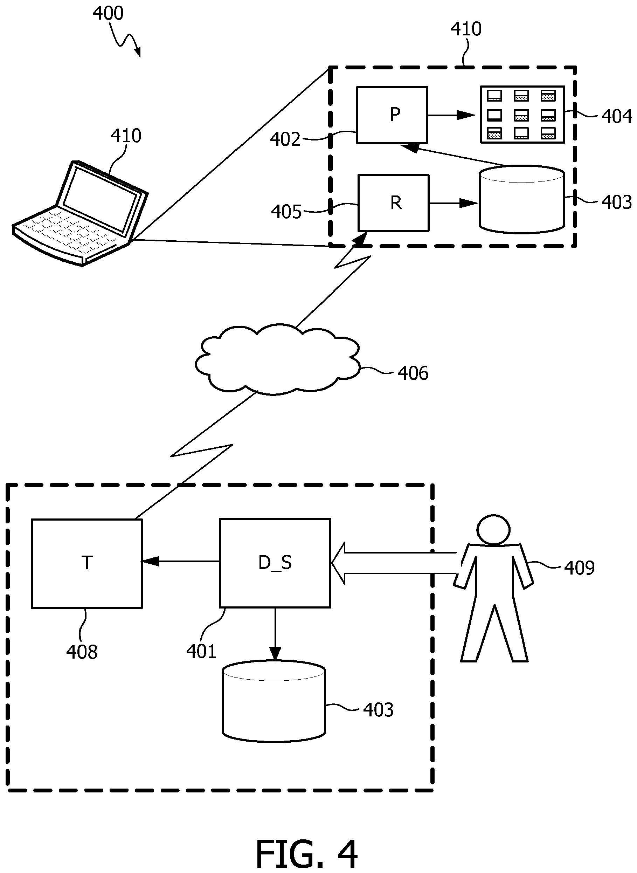

Fig. 4 shows a system 400 used in the method of the present invention for determining a biodistribution 404 of radioactive agents in a subject 409, where the system 400 comprises a detector system (D_S) 401 and a processor (P) 402. The detector system (D_S) 401 comprises one or more detectors from a group of: MOSFET-based detectors adapted to sense gamma radiation in an energy range of 100-550keV, diode-based detectors, Thermal Luminescent Detectors (TLD), film-based detectors and gel-based detectors, and any device adapted to measure such emitted radiation. They may be arranged so that they comprise an integrated circuit and memory chip 403. An example of such a detector is MOSFET-based detectors as disclosed by "P. H. Halvorsen, Medical Physics, vol. 32, pp. 110-117, 2005 ", that further comprises integrated circuit and memory chip 403. The luminescent detector can be based on crystallography memory where the captured radiation data are stored in the crystal and where by heating the crystal the "stored" data are released in a form of emitted light. These sensors may further include a portable memory feature so that they can manually be transferred to a computer device 410, which can e.g. comprise a regular PC computer, a PDA, mobile phone, media player and any type of intelligent devices comprising processor (P) 402 and memory 403.

In one embodiment of the method of the present invention, the system 400 further comprises a transmitter (T) 408 for transmitting the measured data over a communication channel 406 to the computer device 410, where a receiver (R) 405 receives the transmitted data and stores it in the memory 403 such as ROM, RAM, DRAM, SRAM and the like. The processing of the data as discussed previously, i.e. the fitting process where the measured data are initially fitted to determine the time-activity-curve and the subsequent integral calculation is typically performed at the computer device 410. However, the processor (P) 402 and the memory 403 could just as well be arranged on the user side (not shown here), either integrated into the detector system (D_S) 401 or be placed or attached to the subject.

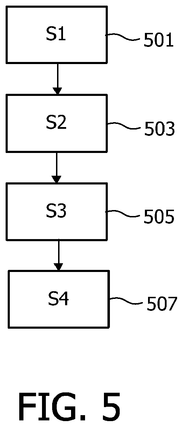

Fig. 5 shows a flowchart of a method according to the present invention of determining a biodistribution of radioactive agents in a subject.

Initially, the radiation emitted from the imaging agents at localized target tissues and/or risk tissues within the subject is measured (S1) 501, wherein the measuring rate is adapted to the pharmacokinetic behavior of the tissues (S2) 503 and result in separate radiation data sets (e.g. as shown in Fig. 2) associated to the tissues.

In one embodiment, adapting the measuring rate to the pharmacokinetic behavior of the tissues comprises adapting the measuring rate to the activity dynamics of the tissues. In another embodiment, this includes using a measuring rate that is the same for all the detectors and is adapted to the tissue having the highest activity dynamics. This is to ensure that the most relevant data points shown in Fig. 2 will be captured, e.g. those around the maximum $A(t)$ value. It is particularly important to adapt the measuring rate to the pharmacokinetic behavior of the tissues so that the steep part of the plot along with part where the emitted radiation reaches its maximum value is captured. The "tail part" of the plot is of course highly relevant but the measuring rate may be lowered, without jeopardizing that relevant information gets lost. A model of the tail, based on measured data could be used.

The data sets are then processed (S3) 505 for determining the biodistribution for each respective tissue.

In one embodiment, the resulting biodistribution is used as an indicator for e.g. the succeeding dose distribution of the radioactive therapeutic agents including alpha-and beta emitting isotopes such as $^{90}$Y in the therapeutic version of the agent Zevalin® or $^{131}$I in the case of the agent Bexxar® within the tissues (S4) 507.

In another embodiment, the resulting biodistribution is used for research and approval of new drugs that have such radiolabels attached, where the biodistribution is used to obtain information how these drugs will be metabolized in the body (S4) 507.

The processing of the data sets includes applying a fitting process on each respective data set (S3) 505 for determining time-activity curve illustrating the biodistribution for each respective tissue over time and subsequently determining the integral of the time-activity curve for each respective data set.

Certain specific details of the disclosed embodiment are set forth for purposes of explanation rather than limitation, so as to provide a clear and thorough understanding of the present invention. However, it should be understood by those skilled in this art, that the present invention might be practiced in other embodiments that do not conform exactly to the details set forth herein, without departing from the scope of the claims. Further, in this context, and for the purposes of brevity and clarity, detailed descriptions of well-known apparatuses, circuits and methodologies have been omitted so as to avoid unnecessary detail and possible confusion.

Reference signs are included in the claims, however the inclusion of the reference signs is only for clarity reasons and should not be construed as limiting the scope of the claims.

## Claims

1. A method of determining a biodistribution of radioactive agents in a subject (409), comprising:

   - measuring (501), at localized areas on the subject, radiation emitted from the radioactive agents at localized tissues within the subject with at least one measuring rate using two or more detectors (310a-310f) attached to the skin of the subject, the measuring resulting in separate radiation data sets associated to the localized tissues, the localized tissues including at least one risk tissue, representing healthy tissue that is in risk of being damaged by the radiation emitted from radioactive agents in a succeeding therapy, and at least one target tissue, representing tissue that is intended to be targeted by radioactive agents in the succeeding therapy, and
   - processing (505) the separate radiation data sets for determining radioactivity within each respective localized tissue from the localized tissues and based thereon the biodistribution of the radioactive agents within the subject,

   **characterized in that** the measuring rate is adapted to pharmacokinetic behavior of the localized tissues (300-305).

2. The method according to claim 1, wherein the determined biodistribution of the radioactive agents is used for estimating (507) a dose distribution of succeeding radioactive therapeutic agents in the subject.

3. The method according to claim 1, wherein the radioactive agents are adapted to be attached or associated to pharmaceuticals so that the biodistribution of the radioactive agents reflect a biodistribution of the pharmaceuticals within the subject (507).

4. The method according to claim 1, wherein adapting (503) the at least one measuring rate to the pharmacokinetic behavior of the localized tissues comprises adapting the at least one measuring rate to activity dynamics of the localized tissues.

5. The method according to claim 1, wherein adapting (503) the at least one measuring rate to the pharmacokinetic behavior of the localized tissues comprises using a single measuring rate that is adapted to the pharmacokinetic behavior of the tissue having the highest activity dynamics.

**6.** The method according to claim 1, wherein processing (505) the data sets comprises:

- applying a fitting process to each respective data set from the separate radiation data sets, the fitting process resulting in a time-activity curve associated to the each respective data set, and subsequently
- determining the integral of the time-activity curve for the each respective data set.

**7.** The method according to claim 1, wherein the measuring is performed until a count rate of the radiation emitted from the radioactive agents has fallen below a pre-defined threshold value.

**Patentansprüche**

**1.** Verfahren zum Bestimmen einer Bioverteilung von radioaktiven Mitteln in einem Subjekt (409), umfassend:

Messen (501), in lokalisierten Bereichen auf dem Subjekt, der Strahlung, die von den radioaktiven Mitteln an lokalisierten Geweben innerhalb des Subjekts emittiert wird, wobei mindestens eine Messgeschwindigkeit von zwei oder mehr Detektoren (310a-310f), die an der Haut des Subjekts befestigt sind, verwendet wird, wobei das Messen in separaten Strahlungsdatensätzen resultiert, die den lokalisierten Geweben zugeordnet sind, wobei die lokalisierten Gewebe mindestens ein Risikogewebe aufweisen, welches für das gesunde Gewebe steht, das in Gefahr ist, durch die Strahlung beschädigt zu werden, die von den radioaktiven Mitteln in einer nachfolgenden Therapie emittiert wird, und mindestens ein Zielgewebe, das durch die radioaktiven Mittel in der nachfolgenden Therapie angezielt werden soll, und
Verarbeiten (505) der separaten Strahlungsdatensätze zum Bestimmen der Radioaktivität innerhalb jedes jeweiligen lokalisierten Gewebes der lokalisierten Gewebe und darauf basierend die Bioverteilung der radioaktiven Mittel innerhalb des Subjekts,
**dadurch gekennzeichnet, dass** die Messgeschwindigkeit an das pharmakokinetische Verhalten der lokalisierten Gewebe (300-305) angepasst ist.

**2.** Verfahren nach Anspruch 1, wobei die bestimmte Bioverteilung der radioaktiven Mittel zum Schätzen (507) einer Dosierverteilung der nachfolgenden radioaktiven therapeutischen Mittel in dem Subjekt verwendet wird.

**3.** Verfahren nach Anspruch 1, wobei die radioaktiven Mittel dazu ausgelegt sind, an Pharmazeutika angehängt oder damit verknüpft zu werden, so dass die Bioverteilung der radioaktiven Mittel eine Bioverteilung der Pharmazeutika innerhalb des Subjekts (507) widerspiegelt.

**4.** Verfahren nach Anspruch 1, wobei das Anpassen (503) der mindestens einen Messgeschwindigkeit an das pharmakokinetische Verhalten der lokalisierten Gewebe das Anpassen der mindestens einen Messgeschwindigkeit an die Aktivitätsdynamik der lokalisierten Gewebe umfasst.

**5.** Verfahren nach Anspruch 1, wobei das Anpassen (503) der mindestens einen Messgeschwindigkeit an das pharmakokinetische Verhalten der lokalisierten Gewebe das Verwenden einer einzigen Messgeschwindigkeit umfasst, die an das pharmakokinetische Verhalten des Gewebes, das die höchste Aktivitätsdynamik aufweist, angepasst ist.

**6.** Verfahren nach Anspruch 1, wobei das Verarbeiten (505) der Datensätze umfasst:

Anwenden eines Bestückungsvorgangs für jeden zugehörigen Datensatz aus den separaten Strahlungsdatensätzen, wobei der Bestückungsvorgang in einer Zeitaktivitätskurve resultiert, die dem jeweiligen Datensatz zugeordnet ist, und nachfolgendes
Bestimmen des Integrals der Zeitaktivitätskurve für jeden zugehörigen Datensatz.

**7.** Verfahren nach Anspruch 1, wobei das Messen durchgeführt wird, bis eine Zählgeschwindigkeit der Strahlung, die von den radioaktiven Mitteln emittiert wird, unter einen vordefinierten Schwellenwert gefallen ist.

**Revendications**

**1.** Procédé de détermination d'une biodistribution d'agents radioactifs chez un sujet (409), comprenant :

la mesure (501), au niveau de zones localisées sur le sujet, d'un rayonnement émis par les agents radioactifs au niveau de tissus localisés au sein du sujet avec au moins un taux de mesure à l'aide de deux détecteurs (310a à 310f) ou plus fixés à la peau du sujet, la mesure aboutissant à des ensembles de données de rayonnement séparés associés aux tissus localisés, les tissus localisés incluant au moins un tissu à risque, représentant un tissu sain qui présente les risques d'être endommagé par des rayonnements émis par des agents radioactifs dans une thérapie suivante, et au moins un tissu cible, représentant un tissu qui est destiné à être ciblé par des agents radioactifs dans la thérapie suivante, et

le traitement (505) des ensembles de données de rayonnement séparés pour déterminer la radioactivité au sein de chaque tissu localisé respectif des tissus localisés et d'après cela, la biodistribution des agents radioactifs au sein du sujet,

**caractérisé en ce que** le taux de mesure est adapté à un comportement pharmacocinétique des tissus localisés (300 à 305).

2. Procédé selon la revendication 1, dans lequel la biodistribution déterminée des agents radioactifs est utilisée pour estimer (507) une distribution de dose d'agents thérapeutiques radioactifs suivants au sujet.

3. Procédé selon la revendication 1, dans lequel les agents radioactifs sont adaptés pour être fixés ou associés à des médicaments de sorte que la biodistribution des agents radioactifs reflète une biodistribution des médicaments au sein du sujet (507).

4. Procédé selon la revendication 1, dans lequel l'adaptation (503) de l'au moins un taux de mesure au comportement pharmacocinétique des tissus localisés comprend l'adaptation de l'au moins un taux de mesure à une dynamique d'activité des tissus localisés.

5. Procédé selon la revendication 1, dans lequel l'adaptation (503) de l'au moins un taux de mesure au comportement pharmacocinétique des tissus localisés comprend l'utilisation d'un taux de mesure unique qui est adapté au comportement pharmacocinétique des tissus ayant la dynamique d'activité la plus élevée.

6. Procédé selon la revendication 1, dans lequel le traitement (505) des ensembles de données comprend :

   - l'application d'un processus d'ajustement à chaque ensemble de données respectif des ensembles de données de rayonnement séparés, le processus d'ajustement aboutissant en une courbe de temps-activité associée à chaque ensemble de données respectif, et ensuite
   - la détermination de l'intégrale de la courbe de temps-activité pour chaque ensemble de données respectif.

7. Procédé selon la revendication 1, dans lequel la mesure est réalisée jusqu'à ce qu'un taux de comptage du rayonnement émis par les agents radioactifs ait chuté en dessous d'une valeur de seuil prédéfinie.

FIG. 1

FIG. 2

FIG. 3

FIG. 4

```
┌─────────────┐
│             │
│     S1      │╮──501
│             │
└──────┬──────┘
       │
       ▼
┌─────────────┐
│             │
│     S2      │╮──503
│             │
└──────┬──────┘
       │
       ▼
┌─────────────┐
│             │
│     S3      │╮──505
│             │
└──────┬──────┘
       │
       ▼
┌─────────────┐
│             │
│     S4      │╮──507
│             │
└─────────────┘
```

# FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02086449 A2

- US 20030125616 A1

**Non-patent literature cited in the description**

- **SGOUROS G.** Dosimetry of Internal Emitters. *J Nucl Med,* 2005, vol. 46, 18-27
- **G. SGOUROS.** *The Journal of Nuclear Medicine,* January 2005, vol. 46 (1), 18s-27s

- **P. H. HALVORSEN.** *Medical Physics,* 2005, vol. 32, 110-117